# EUROPEAN PATENT SPECIFICATION

(11) **EP 1 488 818 B1**
(45) Date of publication and mention of the grant of the patent: **03.03.2010**
(21) Application number: 03744519.4
(22) Date of filing: 11.03.2003
(51) Int. Cl.: A61M 5/315

(54) **PREFILLED SYRINGE WITH PLUNGER BACKWARD MOVEMENT LIMITING MECHANISM**
VORGEFÜLLTE SPRITZE MIT MECHANISMUS ZUR EINSCHRÄNKUNG DER RÜCKWÄRTSBEWEGUNG DES KOLBENS
SERINGUE PREREMPLIE A MECANISME LIMITANT LE MOUVEMENT DU PISTON VERS L'ARRIERE

(30) Priority: 15.03.2002 JP 2002072083
(43) Date of publication of application: 22.12.2004
(73) Proprietor: Becton Dickinson and Company, Franklin Lakes, NJ 07417-1880 (US); Kyowa Hakko Kirin Co., Ltd., Tokyo 100-8185 (JP)
(72) Inventor: HATO, Seiichi, c/o Nippon Becton Dickinson Co.Ltd, Minato-ku, Tokyo 107-0052 (JP)
(74) Representative: Weber, Thomas
(86) International application number: PCT/JP2003/002851
(87) International publication number: WO 2003/077976

(56) References cited:
- JP-A- 3 503 608
- JP-A- 8 229 122
- JP-A- 8 504 352
- US-A- 4 367 738
- US-A- 5 370 621
- US-A- 5 728 075
- US-A- 5 803 918

## Description

### Technical Field

The present invention relates to a syringe in which injection liquid is prefilled (prefilled syringe), and more particularly, the prefilled syringe which is provided with a means for preventing contamination of the prefilled injection liquid

### Background Technique

For a conventional type of prefilled syringe, injection liquid is filled into the syringe barrel and a sliding plug (of the liquid-injecting plunger) is inserted in the barrel, usually under a sterilized condition, and thereafter, a plunger rod is fixed to the sliding plug and works such as labeling and packaging are done for shipment under a non-sterilized condition.

A syringe in which some specific injection liquid is prefilled may be finally sterilized by heating using an autoclave etc. after packaging. However, in other type of syringe in which injection liquid unable to be sterilized by heating is prefilled, bacteria or other microorganisms are considered to have started to adhere to the syringe immediately after it was brought out from the sterilized condition. Namely, in such condition, only the liquid-filled space sealed by the sliding plug (of the plunger) in the barrel is kept sterilized, and there is a possibility that microorganisms adhere to the backward area of the sliding plug, even if the backward area is inside the barrel, before the prefilled syringe is used.

Consequently, in case of the above-mentioned syringe, when the sliding plug is withdrawn backward, there is a risk that bacteria or other microorganisms adhering to the inner surface of the barrel may contaminate injection liquid and the syringe is therefore hazardous. The control of such risk has been depending on the careful handling of the prefilled syringe by care providers such as physicians and nurses, but their mishandling has been reasonably anticipated. However, in reality, injection liquid was contaminated due to the mishandling, that may result in medical malpractice. Even if such contamination of injection liquid and medical malpractice due to it occur, it was difficult to determine that such malpractice was attributable to the mishandling of the prefilled syringe and the problem has therefore not been clearly recognized.

A delivery apparatus in the form of a syringe assembly is disclosed in US-A-5,370,621. This syringe assembly comprises in the interior of a syringe container a lock sleeve having a flange which seats on the rear flange of the syringe container and a tubular portion, which fits snugly within and against the interior surface of the syringe container. The piston rod comprises a stop member spaced below the lower surface of the lock sleeve. Withdrawal of the piston rod is only possible up to the point when the stop member abuts against the lower surface of the lock sleeve. The lock sleeve comprises an outer flange, which seats on a rear flange of the syringe container. With such a structure, the lock sleeve extends to a great extent into the interior of the syringe container. It may be secured by an adhesive or a tight friction fit.

US 4 367 738 discloses a delivery apparatus having a flange and a piston rod having spikes. The withdrawal of the plunger rod from the barrel is made impossible by the abutment of the spikes on the flange when the user tries to pull it out.

It is an object of the invention to provide a prefilled syringe entirely free from a risk of injection liquid contamination due to mishandling and resultant medical malpractice.

### DISCLOSURE OF THE INVENTION

The prefilled syringe of the invention is defined by Claim 1. Accordingly, it is **characterized in that** said plunger withdrawal-limiting member is an annular withdrawal-limiting flange which is provided at the rear end of the opening of said barrel and extends inwardly in the radial direction from the periphery of said rear end opening of the barrel.

The invention provides the prefilled syringe comprising a barrel having a closed front-end wall with a small passageway through which injection liquid is discharged and a rear opening, a plunger including a sliding plug liquid-tightly contacting with the inner surface of the barrel and partitioning the inside of the barrel into a liquid-filled area which is formed between the plunger and the closed front-end wall and in which injection liquid is filled and sealed and a liquid-unfilled area in the back of the liquid-filled area and a plunger rod extending backward from the sliding plug through the rear opening of the barrel, and a plunger withdrawal-limiting member fixed to the barrel, which limits withdrawal of the plunger by engaging the plunger and prevents injection liquid from contacting with the surface of the liquid-unfilled area in the barrel.

Referring more particularly, the plunger withdrawal-limiting member which is provided at the rear opening of the barrel and extends from the rear opening to the inside of the barrel for a certain length, can contact with the backward-moving sliding plug of the plunger so that withdrawal of the plunger is limited.

It is also possible that the plunger withdrawal-limiting member provided at the rear opening of the barrel is in the form of an annular withdrawal-limiting flange comprising an opening for the plunger rod which extends inwardly in the radial direction from the periphery of the rear opening of the barrel and is sized to allow the plunger rod to pass therethrough, while a stopper member extending outwardly in the radial direction from the plunger rod is provided. As a result of this, withdrawal of the plunger is limited by engagement of the stopper member with the withdrawal-limiting flange.

In the above case, it is possible to stably push and handle the plunger by allowing at least a portion of the outer peripheral surface of the withdrawal-limiting member to closely contact with the inner peripheral surface of the barrel. It is also possible that the position in which the withdrawal-limiting member is fixed to the plunger rod is adjusted at needs in accordance with the quantity of injection liquid to be prefilled.

In other form of embodiment, the withdrawal-limiting member may be made in the form of a cylindrical member in which a male screw is provided on the outer peripheral surface thereof, and on the other hand, the plunger withdrawal-limiting member may be formed in the form of an annular flange in which the inner peripheral surface of the flange defines an opening having a size corresponding to the plunger withdrawal-limiting member, the annular flange being provided at the rear end opening of the barrel and extending inwardly in a radial direction from the peripheral edge of the opening and a female screw to be mated with the male screw is formed on the inner peripheral surface of the flange. In this form of syringe, when the plunger is withdrawn for a predetermined distance over a predetermined position, the stopper member engages the withdrawal-limiting member, thus preventing withdrawal of the plunger. If the male screw of the stopper member is further turned and fitted into the female screw on the plunger withdrawal-limiting member the plunger can be further withdrawn.

Additionally, it is possible that the stopper member is formed in the form of a leaf spring shaped member which extends diagonally backward from a predetermined location in the plunger rod so that when the plunger rod is inserted into the barrel through the opening for the plunger rod the leaf spring is deflected by the surface of the opening for the plunger rod and after passing through the same opening the leaf spring returned to its original shape to act the function to limit plunger withdrawal.

Furthermore, it is possible that the stopper member is formed in a member projecting radially outward from the plunger rod and a notch through which the stopper member passes may be formed at the opening of the withdrawal-limiting flange so that the function of plunger withdrawal can be released.

### Brief Explanation of the Drawing

Fig. 1 is a sectional side elevational view of a prefilled syringe arrangement not belonging to the invention;
Fig. 2 is a sectional side elevational view of a prefilled syringe arrangement in accordance with the second embodiment of the invention;
Fig. 3 is a sectional side elevational view of a prefilled syringe arrangement in accordance with the third embodiment of the invention;
Fig. 4 is a schematic illustration view of a prefilled syringe arrangement in accordance with the fourth embodiment of the invention;
Fig. 5 is a schematic illustration view of a prefilled syringe arrangement in accordance with the fifth embodiment of the invention; and
Fig. 6 is a schematic illustration view of the prefilled syringe according to the sixth embodiment of the invention in which the side view of the withdrawal-limiting flange is also shown.

### Best Mode for Carrying out the Invention

The mode for carrying out the prefilled syringe according to the invention is explained below, referring to the attached drawings.

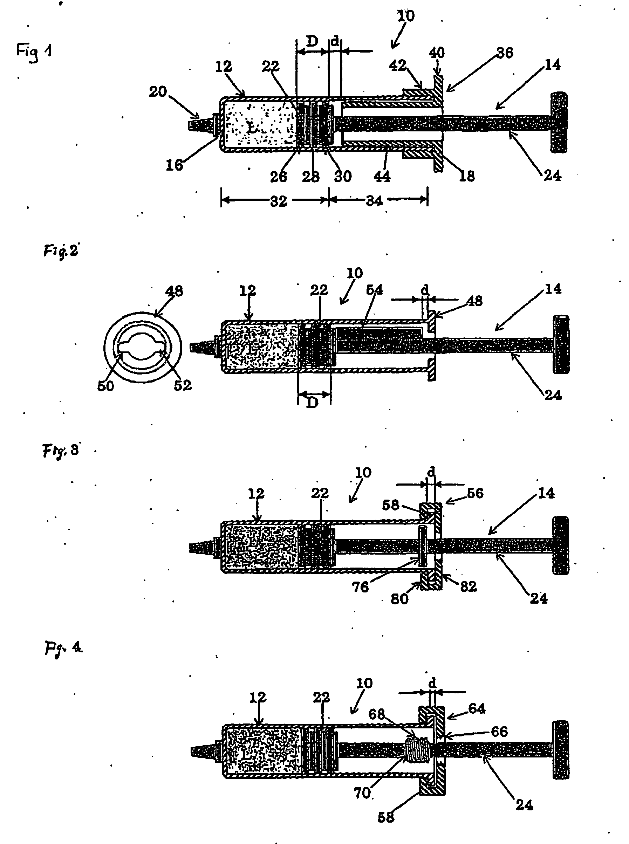

Fig. 1 shows a prefilled syringe 10 not belonging to the invention. As illustrated, the prefilled syringe 10 has a barrel 12 in a cylindrical form and the plunger 14 to be inserted into the barrel. The barrel has the front-end wall 16, the tip of which has a small passageway (not shown in the Fig. 1) through which the injection liquid is discharged, and the rear opening 18 through which the plunger 14 is received. A barrel tip cover 20 is mounted on the outer surface of the tip of the front-end wall 16. The plunger 14 has a sliding plug 22 at its tip which is able to slidably move among the inner surface of the barrel 12 and a plunger rod 24 which extends outwardly from the sliding plug 22 to the outside of the barrel through the rear opening 18 at the rear end of the barrel. Fig. 1 shows the completed prefilled syringe before use. In this state, the sliding plug 22 partitions the inside of the barrel 12 into a liquid-filled area 32 where the injection liquid L is filled and sealed and a liquid-unfilled area 34 in the back of the area 32. In the illustrative embodiment shown in the Fig. 1, the sliding plug 22 has a generally cylindrical form and three ring-shaped ribs 26, 28 and 30 are provided around the outer surface of the sliding plug with Keeping predetermined spaces in the axial direction therebetween. As the ribs liquid-tightly contact with the inner surface of the barrel 12, so that the liquid-filled area 32 in which the injection liquid is filled and sealed is formed between the front-end wall 16 of the barrel and the sliding plug 16. Namely, in this state, the liquid-filled area 32 is formed between the front-end wall 16 and the right side rib 30 and the liquid-unfilled area 34 is formed between the right side rib 30 and the rear opening 18 of the barrel.

As illustrated in the Fig. 1, a withdrawal-limiting member 36 to limit withdrawal of the plunger 14 is provided around the rear opening 18 of the barrel. The withdrawal-limiting member has a base plate 40 in an annular form, which is abutted to the end edge of the rear opening 18, a outer cylindrical portion 42 which extends from the base plate toward the tip of the barrel along the outer surface of the barrel and an inner cylindrical portion 44 which extends longer than the outer cylindrical portion 42 from the base plate toward the tip of the barrel around the inner surface of the barrel. These outer and inner cylindrical portions are secured to the barrel by pinching the side wall of the barrel.

The front-end of the inner cylindrical portion 44 is spaced from the rear end of the sliding plug 22 for a distance of d. Therefore, the sliding plug 22 is able to move rearward by the distance d from the predetermined position shown in Fig.1 and when the sliding plug is moved rearward for the distance d it abuts or engages the front- end of the inner cylindrical portion 44 of the withdrawal-limiting member 36, thus further withdrawal being prevented. The distance d is determined to be shorter than a distance D between the front-end of the sliding plug (more accurately, the position where the left side rib 26 liquid-tightly contacts with the inner surface of the barrel)and the position where the rear end of the right side rib 30 liquid-tightly contacts with the inner surface of the barrel so that when the sliding plug is withdrawn no contact between injection liquid L and the inner surface in the liquid-unfilled area in the barrel.

For the above-mentioned prefilled syringe, the plunger 14 and the withdrawal-limiting member 36 may be formed from a general-purpose resin such as polypropylene and polyethylene using injection molding and the sliding plug 22 is preferably be made from rubber or similar materials.

Fig. 2 shows a mode for carrying out the prefilled syringe 10 according to the invention.

In this type of prefilled syringe, an annular withdrawal-limiting flange 48 is integrally molded with the cylindrical barrel 12 in the rear end therefore and an opening through which the plunger rod 24 passes is provided in the central portion of the withdrawal-limiting flange 48. In addition, a pair of notches 50 and 52 are formed diametrically in the diameter of the inner peripheral edge of the flange 48. On the other hand, on the surface of the plunger rod 24, a withdrawal-limiting member 54 extends backward from the rear-end of the sliding plug 22. The withdrawal-limiting member 54 has a shape and a size corresponding to that of the notches 50 and 52 so that the member 54 is able to pass through the notches. Therefore, after injection liquid is prefilled and the sliding plug 22 is loaded, the plunger rod 24 is inserted into the barrel with the withdrawal-limiting part 54 passing through the notch 50 or 52, and then the front-end of the plunger rod can be connected to the sliding plug 22. Between the rear-end of the withdrawal-limiting member 54 and the inner surface of the withdrawal-limiting flange 48, the space d keeping the same relation of D>d as shown in the embodiment in Fig. 1 is provided.

Fig. 3 shows the second mode for carrying out the prefilled syringe according to the invention.

In this type of prefilled syringe, different from the prefilled syringe shown in Fig. 2, the withdrawal-limiting flange 56 is separately formed from the barrel 12 and fixed to a flange 58 formed radially and outwardly of and integrally with the rear end of the barrel. It is possible that in the same manner as the flange holding members disclosed in the Patent No. 2,814,983 and No. 2,845,426, etc., the withdrawal-limiting flange 56 may be an annular form comprising a front annular plate 80 and a rear annular plate 82 between which a space for holding the flange 58 of the barrel is provided. The withdrawal-limiting flange 56 is secured to the barrel in the following manner. Firstly, an aperture (not shown in Fig. 3) extending in radial direction is provided in a portion of the annular form. The withdrawal-limiting flange 56 is then positioned at right angle to the axis of the barrel 12 with the aperture being aligned with the flange 58 of the barrel, and the flange 56 is pushed onto the aperture and then the flange 58 of the barrel and the plunger rod 24 are passed through the aperture into the inside of the withdrawal-limiting flange 56 so that the flange 58 of the barrel is held in the space between the front plate 80 and the rear plate 82

The withdrawal-limiting member 76 in a disc form is fixed to the plunger rod 24 at the distance of d from the rear-end annular plate 82 of the withdrawal-limiting flange 56. In this mode of the invention, by fixing the withdrawal-limiting member at a proper location in the axial direction of the plunger rod 24 depending on the quantity of the injection liquid to be filled in the prefilled syringe, it is possible to provide the withdrawal-limiting member 76 at the distance of d from the rear-end annular plate 82 of the withdrawal-limiting flange. The withdrawal-limiting member 76 in this mode according to the invention may be in a fan shape as ell as in a disc shape, and in either case, it is possible to forward the plunger 14 into the barrel 12 in a stable manner by keeping the outer periphery of the withdrawal-limiting member close to the inner surface of the barrel.

Fig. 4 shows the third mode for carrying out the prefilled syringe according to the invention.

In this type of prefilled syringe 10, a withdrawal-limiting flange 64 similar to the withdrawal-limiting flange 56 shown in Fig. 3. is mounted on the flange 58 which is integrally molded with the rear-end of the barrel 12 and radially extends. The difference of this type of prefilled syringe from that shown in Fig. 3 is that a female screw 66 is formed in the inner periphery of the withdrawal-limiting flange 64 and a male screw 68 fitting into the female screw 64 is correspondingly formed on a withdrawal-limiting member 70 which is fixed to the plunger rod 24.

Between the rear-end of the withdrawal-limiting member 70 and the front surface of the rear annular plate of the withdrawal-limiting flange 64, a space d is provided with keeping the same relation of D>d as shown in the mode in Fig. 1 and when the plunger 14 withdraws from a predetermined position by the distance d, the withdrawal-limiting member 70 abuts the withdrawal-limiting flange 64 to prevent further withdrawal. In order to further withdraw the plunger 14 and draw out the same from the barrel 12, the male screw of the withdrawal-limiting member 70 is turned and fitted into the female screw 66 of the withdrawal-limiting flange.

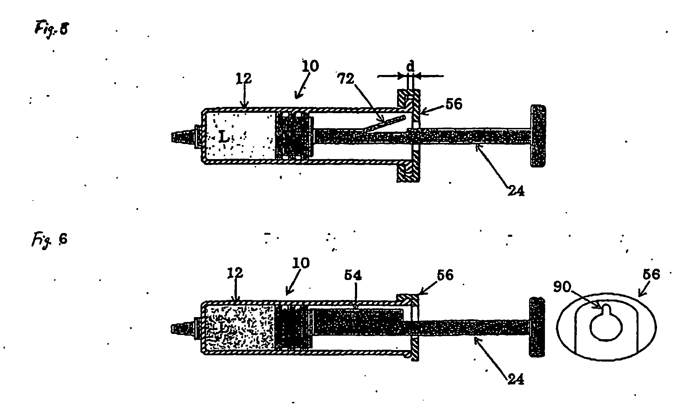

Fig. 5 shows the fourth mode for carrying out the prefilled syringe according to the invention. This type of prefilled syringe 10 is similar to that shown in Fig. 3, but a withdrawal-limiting member 72 fixed to the plunger rod 24 is in the form of leaf spring projecting diagonally backward. That is, when the plunger rod 24 is passed into to the inside of the barrel through the circular opening formed in the withdrawal-limiting flange 56, the leaf spring of the withdrawal limiting member 72 is deflected by the edge of the opening so that the leaf spring of the withdrawal-limiting member 72 passes through the opening into the barrel. After passage into the barrel, the leaf spring of the withdrawal-limiting member is sprung back to the form projecting diagonally backward, as illustrated in the Fig. 5. In this state, when the plunger withdraws from the predetermined position by the distance d, the rear-end of the leaf spring of the withdrawal-limiting member 72 abuts the withdrawal-limiting flange 56 to prevent further withdrawal.

The prefilled syringe shown in Fig. 6 is substantially identical to that shown in Fig. 2, but it is different from the syringe in Fig. 2 in the fact that only one notch 90 is formed in the opening for the plunger rod passage in the withdrawal-limiting flange 56 (shown as oval-shaped in Fig. 6) . The withdrawal-limiting member 54 fixed to the plunger rod 24 can pass through the notch 90 so that the plunger rod 24 is moved forward into the inside of the barrel 12 and is withdrawn from the barrel.

### Industrial Applicabilty

The prefilled syringe according to the invention has the above-mentioned constitution and through its simple structure surely prevents accidental withdrawal of the plunger due to mishandling. If the plunger is inadvertently withdrawn beyond the predetermined position, the injection liquid sealed in the liquid-filled area in the barrel may contact with the surface of the liquid-unfilled area (which bacteria etc. possibly adhere to), resulting in a risk of contamination. The invention can totally avoid such a risk and therefore prevent the occurrence of medical malpractice attributable to the contamination.

To prevent the contamination caused by bacteria, conventionally, the method involving heating sterilization using autoclave etc. has been employed after packing the produced prefilled syringe. However, it is impossible to apply the heating sterilization to some injection liquids and for the syringe prefilled with such liquids, the risk of contamination of such injection liquid is unavoidable. Therefor, the invention is especially beneficial to such prefilled syringe.

Also for the prefilled syringe in which an injection liquid suitable for heating sterilization is sealed, the heating sterilization process involves incremental facility investment and production cost. In this regard, the invention makes it possible to provide users with safer prefilled syringes not needing such costly process and is extremely beneficial to them.

## Claims

1. A prefilled syringe comprising:
a barrel (10) having a closed front-end wall (16) with a small passageway through which injection liquid is discharged and a rear opening (18);
a plunger (14) including a sliding plug (22) liquid tightly contacting with the inner surface of the barrel and partitioning the inside of the barrel into a liquid-filled area (32) which is formed between said plunger and said closed front-end wall and in which the injection liquid is filled and sealed, and a liquid-unfilled area (34) in the back of the liquid-filled area and a plunger rod (24) extending backward from said sliding plug (22) through said rear opening (18) of the barrel; and
a plunger withdrawal-limiting member in the form of a flange (48, 56, 64) fixed to the rear opening of said barrel (10) and extending inwardly in the radial direction from the periphery of said rear end opening (18) of the barrel, which limits plunger withdrawal by engaging said plunger, wherein said plunger withdrawal-limiting member has an opening for said plunger rod (24) extending inwardly in the radial direction and being sized to allow said plunger rod to pass therethrough, and said plunger rod (14) has a stopper member (54,70,72,76) extending outwardly in the radial direction from the plunger rod, withdrawal of said plunger being limited by contact of said stopper member with said withdrawal-limiting member,
**characterized in that**
said flange prevents the injection liquid from contacting with the surface of said liquid-unfilled area (34) in the barrel, and that the distance D between a portion (26) on the outer peripheral surface of the sliding plug (22) which is nearest to the closed front-end wall (16) of said barrel and a portion (30) on the same which is farthest from the closed front-end wall of the barrel and the distance d between the predetermined position of said plunger (14) and the position where said plunger is withdrawn from said predetermined position and then engages said flange (48,56,64) so that withdrawal of said plunger is prevented are adapted to keep the formula D>d.

2. The prefilled syringe according to Claim 1, wherein at least a portion of the outer peripheral surface of said stopper member (76) is designed to make a close contact with the inner peripheral surface of said barrel (10).

3. The prefilled syringe according to one of Claims 1 or 2, wherein said stopper member (76) is in the form of a plate provided at right angles to the axis of the plunger rod (24) and is able to be fixed to a required position in the axial direction of said plunger rod.

4. The prefilled syringe according to Claim 1, wherein said stopper member (70) is in the form of a cylindrical member having a male screw (68) provided on the outer peripheral surface thereof, and a female screw (66) mating with said male screw is formed at the opening for the plunger rod passage provided in said withdrawal-limiting flange.

5. The prefilled syringe according to Claim 1, wherein said stopper member (72) is a leaf spring extending diagonally backward from a predetermined position in said plunger rod (24), said leaf spring is adapted to be deflected by the surface of said opening for the plunger rod passage to be passed through the same opening when the plunger rod is inserted into said barrel through the same opening and is returned to an original shape after passing through the same opening.

6. The prefilled syringe according to Claim 1, wherein said stopper member (54) projects radially outward from said plunger rod (24) and a notch (90) for allowing said stopper member to pass through is formed at said opening for the plunger rod passage in said withdrawal-limiting flange (56).

## Patentansprüche

1. Vorbefüllte Spritze mit:
einem Zylinder (10) mit einer geschlossenen Vorderendwand (16), die einen kleinen Durchlass aufweist, durch welchen Injektionsflüssigkeit ausgegeben wird, und mit einer hinteren Öffnung (18);
einem Kolben (14) mit einem Gleitstopfen (22), welcher in flüssigkeitsdichtem Kontakt mit der Innenfläche des Zylinders steht und das Innere des Zylinders in einen flüssigkeitsgefüllten Bereich (32), welcher zwischen dem Kolben und der geschlossenen Vorderendwand gebildet ist und in welchen die Flüssigkeit gefüllt und dicht gehalten ist, und einen nicht flüssigkeitsgefüllten Bereich (34) hinter dem flüssigkeitsgefüllten Bereich unterteilt, und mit einer Kolbenstange (24), die sich von dem Gleitstopfen (22) nach hinten durch die hintere Öffnung (18) des Zylinders erstreckt; und
einem Kolbenrückzugsbegrenzungselement in Form eines Flanschs (48, 56, 64), der an der hinteren Öffnung des Zylinders (10) befestigt ist und sich vom Umfang der hinteren Endöffnung (18) des Zylinders radial nach innen erstreckt, welches den Rückzug des Kolbens durch Angreifen an dem Kolben begrenzt, wobei das Kolbenrückzugsbegrenzungselement eine Öffnung für die Kolbenstange (24) aufweist, die sich radial nach innen erstreckt und derart bemessen ist, dass sie den Durchtritt der Kolbenstange ermöglicht, und die Kolbenstange (24) ein Anschlagteil (54, 70, 72, 76) aufweist, das sich von der Kolbenstange radial nach außen erstreckt, wobei der Rückzug des Kolbens durch den Kontakt zwischen dem Anschlagteil und dem Rückzugsbegrenzungselement begrenzt wird,
**dadurch gekennzeichnet, dass**
der Flansch den Kontakt der Injektionsflüssigkeit mit der Oberfläche des nicht flüssigkeitsgefüllten Bereichs (34) in dem Zylinder verhindert, und dass der Abstand D zwischen einem Bereich (26) auf der Außenumfangsfläche des Gleitstopfens (22), welcher der geschlossenen Vorderendwand (16) des Zylinders am nächsten ist, und einem Bereich (30) auf demselben, welcher von der geschlossenen Vorderendwand des Zylinders am weitesten entfernt ist, und der Abstand d zwischen der vorbestimmten Position des Kolbens (14) und der Position, in welcher der Kolben von der vorbestimmten Position zurückgezogen ist und an dem Flansch (48, 56, 64) angreift, so dass der Rückzug des Kolbens verhindert ist, so gewählt sind, dass sie die Formel D>d einhalten.

2. Vorbefüllte Spritze nach Anspruch 1, bei welcher zumindest ein Bereich der Außenumfangsfläche des Anschlagteils (76) derart ausgebildet ist, dass er in engem Kontakt mit der Innenumfangsfläche des Zylinders (10) steht.

3. Vorbefüllte Spritze nach Anspruch 1 oder 2, bei welcher das Anschlagteil (76) in Form einer Platte vorgesehen ist, die im rechten Winkel zur Achse der Kolbenstange (24) vorgesehen ist und die an einer erforderlichen Position in axialer Richtung der Kolbenstange befestigbar ist.

4. Vorbefüllte Spritze nach Anspruch 1, bei welcher das Anschlagteil (70) in Form eines zylindrischen Teils mit einem Außengewinde (68) auf dessen Außenumfangsfläche, und wobei ein mit dem Außengewinde zusammengreifendes Innengewinde (66) an der im Rückzugsbegrenzungsflansch vorgesehenen Öffnung für den Durchtritt der Kolbenstange ausgebildet ist.

5. Vorbefüllte Spritze nach Anspruch 1, bei welcher das Anschlagteil (72) eine Blattfeder ist, welche sich diagonal von einer vorbestimmten Position in der Kolbenstange (24) nach hinten erstreckt, wobei die Blattfeder durch die Oberfläche der Kolbenstangendurchtrittsöffnung auslenkbar ist, um durch die selbe Öffnung hindurchgeführt zu werden, wenn der Kolben durch die selbe Öffnung in den Zylinder eingeführt wird, und wobei sich die Feder nach dem Durchtritt durch die Öffnung in die ursprüngliche Form zurückstellt.

6. Vorbefüllte Spritze nach Anspruch 1, bei welcher das Anschlagteil (54) von der Kolbenstange (24) radial nach außen vorsteht und eine den Durchtritt des Anschlagteils erlaubende Kerbe (90) an der Kolbenstangendurchtrittsöffnung in dem Rückzugsbegrenzungsflansch (56) ausgebildet ist.

## Revendications

1. Séringue préremplie comprenant:
un cylindre (10) comprenant une paroi de l'extrémité avant fermée (16) avec un petit passage à travers duquel est déchargée une liquide d'injection, et une ouverture arrière (18);
un poussoir (14) comprenant un bouchon glissant (22) en contact étanche à liquide avec la face intérieure du cylindre et sectionnant l'intérieur du cylindre en une zone remplie de liquide (32), formée entre ledit poussoir et ladite paroi de l'extrémité avant fermée et dans laquelle la liquide d'injection est versée et retenue de manière étanche, et en une zone non remplie de liquide (34) à l'arrière de la zone remplie de liquide, et une tige du poussoir (24) s'étendant vers l'arrière à partir du bouchon glissant (22) à travers ladite ouverture (18) du cylindre; et
un élément à limiter le mouvement en arrière du poussoir en forme d'un flanc (48, 56, 64) fixé à l'ouverture arrière du cylindre (10) et s'étendant radialement vers l'intérieur depuis la périphérie de l'ouverture d'extrémité arrière (18) du cylindre, lequel élément limite le mouvement en arrière du poussoir en engageant ledit poussoir, ledit élément à limiter le mouvement en arrière du poussoir comprenant une ouverture pour ladite tige de poussoir (24) s'étendant radialement vers l'intérieur et étant dimensionnée pour permettre le passage de ladite tige du poussoir à travers celle-ci, et ladite tige de poussoir (24) comprend un élément d'arrêt (54, 70, 72, 76) s'étendant radialement vers l'extérieur depuis la tige du poussoir, le mouvement en arrière du poussoir étant limité par contact dudit élément d'arrêt avec ledit élément à limiter le mouvement en arrière,
**caractérisé en ce que**
ledit flanc empêche le contact de la liquide d'injection avec la surface de ladite zone non remplie de liquide (34) dans le cylindre, et que la distance D entre une partie (26) sur la périphérie externe du bouchon glissant (22) la plus proche à ladite paroi de l'extrémité avant fermée (16) dudit cylindre et une partie (30) sur le même qui est la plus éloignée de ladite paroi de l'extrémité avant fermée, et la distance d entre la position prédéterminée dudit poussoir (14) et la position, dans laquelle le poussoir est retiré de cette position prédéterminée et contacte ledit flanc (48, 56, 64) de manière que le mouvement en arrière du poussoir est bloqué, sont aptes à respecter la formule D>d.

2. Séringue préremplie selon la revendication 1, dans laquelle au moins une partie de la face périphérique externe dudit élément d'arrêt (76) est configurée pour faire un contact intime avec la face périphérique interne du cylindre (10).

3. Séringue préremplie selon l'une des revendications 1 ou 2, dans laquelle ledit élément d'arrêt (76) est en forme d'une plaque prévue en angle droit par rapport à l'axe de la tige du poussoir (24) et est apte à être fixée à une position nécessaire selon la direction axiale de la tige du poussoir.

4. Séringue préremplie selon la revendication 1, dans laquelle ledit élément d'arrêt (70) est en forme d'un élément cylindrique avec un filetage mâle (68) prévu sur sa face périphérique externe, et un filetage femelle (66) coopérant avec le filetage mâle est formé à ladite ouverture pour le passage de ladite tige du poussoir formée dans ledit flanc à limiter le mouvement en arrière.

5. Séringue préremplie selon la revendication 1, dans laquelle ledit élément d'arrêt (72) est un ressort à lames s'étendant diagonalement vers l'arrière à partir d'une position prédéterminée dans ladite tige de poussoir (24), ledit ressort à lames étant apte à être déflêchi par la surface de ladite ouverture pour le passage de ladite tige du poussoir pour être passé à travers ladite ouverture lorsque la tige du poussoir est insérée dans ledit cylindre à travers la même ouverture et il rappelle sa forme originale après avoir passé ladite ouverture.

6. Séringue préremplie selon la revendication 1, dans laquelle ledit élément d'arrêt (54) est en saillie radialement vers l'extérieur depuis ladite tige de poussoir (24) et une encoche (90) permettant le passage dudit élément d'arrêt est formée à ladite ouverture de passage de ladite tige de poussoir dans le flanc à limiter le mouvement en arrière (56).
